(19) Europäisches Patentamt
European Patent Office
Office européen des brevets

(11) Publication number : 0 512 779 A1

# (12) EUROPEAN PATENT APPLICATION

(21) Application number : 92304005.9

(51) Int. Cl.[5] : A61K 31/365, A61K 31/70

(22) Date of filing : 01.05.92

(30) Priority : 02.05.91 US 694720

(43) Date of publication of application :
11.11.92 Bulletin 92/46

(84) Designated Contracting States :
AT BE CH DE DK ES FR GB GR IT LI LU NL PT SE

(71) Applicant : ELI LILLY AND COMPANY
Lilly Corporate Center
Indianapolis Indiana 46285 (US)

(72) Inventor : Matsuoka, Tats
1517 Brunner Drive
Greenfield, Indiana 46140 (US)
Inventor : McClary, David Glen
3804 South White Oak Drive
New Palestine, Indiana 46163 (US)
Inventor : Ose, Earl Eugene
805 Oak Court, R.R. 7
Greenfield, Indiana 46140 (US)
Inventor : Thomson, Thomas Donald
105 Jefferson Blvd.
Greenfield, Indiana 46140 (US)

(74) Representative : Tapping, Kenneth George et al
Erl Wood Manor
Windlesham Surrey, GU20 6PH (GB)

## (54) Tilmicosine and related compounds for the production of a medicament for the treatment of mastitis.

(57) The invention is directed to the use of tilmicosin and other compounds of U.S. Patent 4,820,695 for the treatment of mastitis in nonlactating ruminants such as dairy cattle.

EP 0 512 779 A1

This invention relates to the treatment or prevention of mastitis in ruminants.

Mastitis, an inflammation of the udder, is a troublesome problem, especially of the dairy industry. It is caused by an infection, by any of a number of different pathogens, including Staphylococcus sp., Streptococcus sp. and others. However, there are difficulties in treating mastitis. One useful method is "dry cow" therapy, that is, treatment of the cow at the time of drying off when milking is stopped. The only recognized route of treatment of the dry cow is intramammary infusion of an antibiotic through the teats. However, dispersion of an antibiotic through the mammary gland following intramammary infusion may be limited. In addition, this route of treatment has the risk of introducing a mastitis causing pathogen. Systemic administration of medicaments has been considered; however, the problem is then one of obtaining sufficient levels of the medicament in the mammary gland to be efficacious.

Surprisingly, the applicants have now discovered that mastitis can be effectively treated by the use of the compounds described in U.S. Patent 4,820,695. This patent describes certain derivatives of macrolide antibiotics. One of these has been named "tilmicosin" by USAN, the United States Adopted Names organization. Tilmicosin and other compounds of U.S. Patent 4,820,695 can be employed to treat mastitis.

Thus, according to one aspect of the present invention, there is provided a method of treating or preventing mastitis which comprises subcutaneously administering a compound of formula I

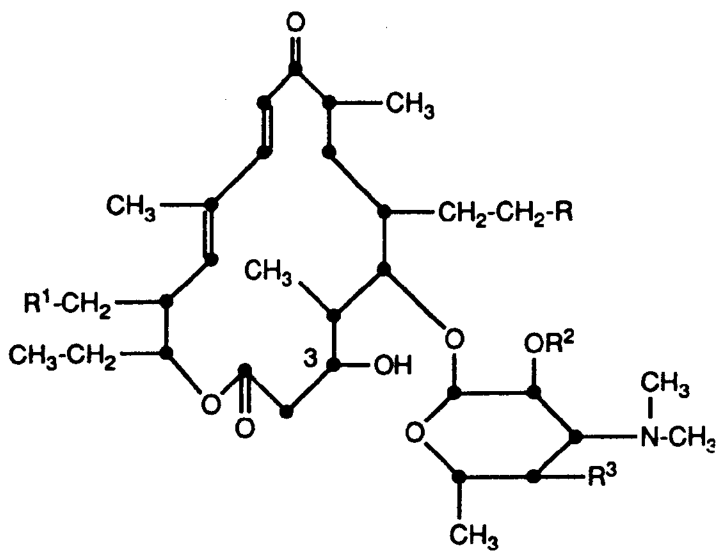

wherein

R is a saturated or unsaturated secondary amino group of the formula

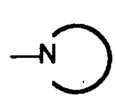

in which the nitrogen atom is part of an otherwise carbocyclic ring system selected from a monocyclic ring containing from 5 to 16 ring atoms or a bicyclic or tricyclic ring system containing from 8 to 20 ring atoms or such a group wherein one or more of the carbon atoms is substituted by $C_1$-$C_4$ alkyl, $C_2$-$C_4$ alkenyl, $C_2$-$C_4$ alkynyl, $C_1$-$C_4$ alkoxy, $C_1$-$C_4$ alkoxycarbonyl, hydroxyl, $C_1$-$C_4$ alkanoyloxy, halo, halo-$C_1$-$C_4$ alkyl, -N($C_1$-$C_4$ alkyl)2, -N$(CH_2)_m$,

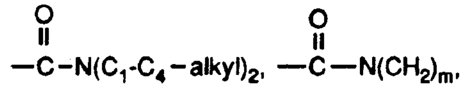

cyano, ethylenedioxy, benzyl, phenyl, or phenyl substituted by from 1 to 3 substituents selected from nitro, halo, $C_1$-$C_4$-alkyl, $C_1$-$C_4$ alkoxy, hydroxy, amino, or mono- or di-($C_1$-$C_4$ alkyl)amino;

m is an interger from 4 through 7;
$R^1$ is

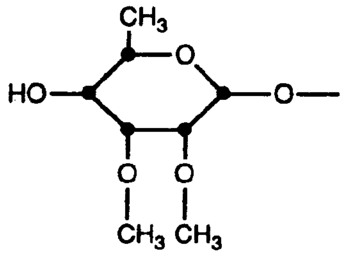 ; 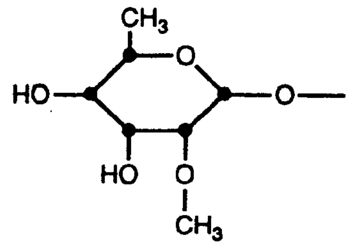 ;

or

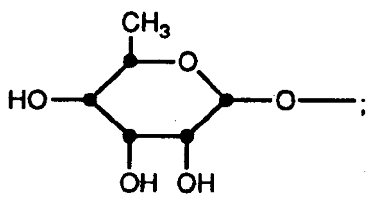 ;

$R^2$ is hydrogen; $C_1$-$C_5$-alkanoyl or $C_1$-$C_5$-alkanoyl having from one to three halo substituents; benzoyl, phenylacetyl or phenylpropionyl or benzoyl, phenylacetyl or phenylpropionyl having from one to five halo or methyl or from one to two methoxyl, nitro or hydroxyl substituents;

$R^3$ is hydroxy; $C_1$-$C_5$ alkanoyloxy; $C_1$-$C_5$-alkanoyloxy having from one to three halo substituents; benzoyloxy, phenylacetoxy or phenoxyacetoxy or benzoyloxy, phenylacetoxy or phenoxyacetoxy having from one to five halo or methyl or from one to two methoxyl, nitro or hydroxyl substituents; or

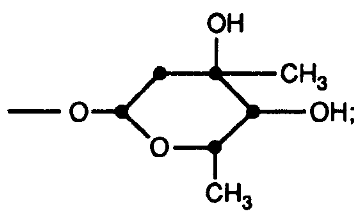

or an acid addition salt thereof;
to a pregnant female ruminant, not less than 25 days prior to parturition, provided that if the pregnant female ruminant has been lactating she is thereafter maintained until parturition without further milking.

This invention can be utilized with any ruminant but is especially useful for cattle. In one embodiment of the present invention, administration occurs as the animal is lactating but will thereafter be managed as a dry cow with no further milking until the next parturition. In another embodiment, a nonlactating animal such as a nulliparous heifer is treated in the period prior to parturition.

The compound of formula I is administered subcutaneously. The amount to be employed is not critical, so long as the amount is effective to control the particular pathogens responsible for the mastitis. In general, amounts of from 0.5 to 10 milligrams per kilogram of body weight will achieve the desired control of mastitis; under many conditions, amounts of from 2 to 8 milligrams per kilogram of body weight are appropriate. An effective amount can be supplied by multiple dosings; but there is no advantage to this practice and a single administration is therefore preferred.

The compound of formula I is desirably formulated for administration. The art of veterinary formulation is well advanced. U.S. Patent 4,820,695 teaches a number of formulations. Example 1 below teaches a preferred formulation.

Thus, in a second aspect of the invention there is provided the use of a compound of formula I

I 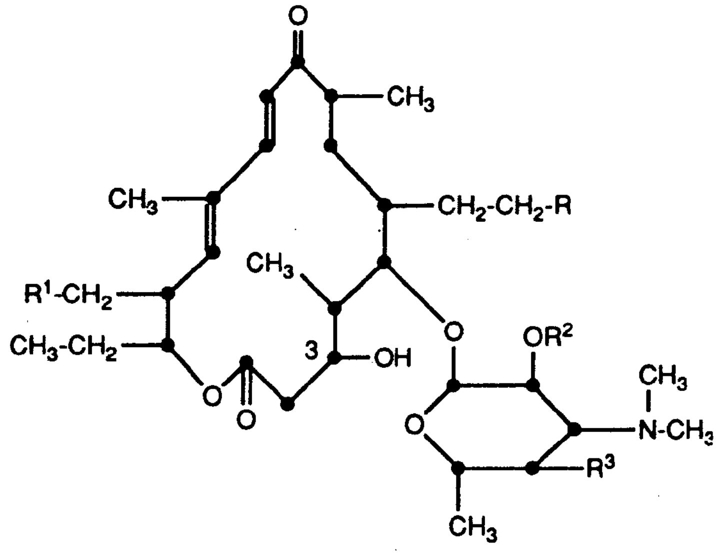

wherein

R is a saturated or unsaturated secondary amino group of the formula

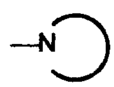

in which the nitrogen atom is part of an otherwise carbocyclic ring system selected from a monocyclic ring containing from 5 to 16 ring atoms or a bicyclic or tricyclic ring system containing from 8 to 20 ring atoms or such a group wherein one or more of the carbon atoms is substituted by $C_1$-$C_4$ alkyl, $C_2$-$C_4$ alkenyl, $C_2$-$C_4$ alkynyl, $C_1$-$C_4$ alkoxy, $C_1$-$C_4$ alkoxycarbonyl, hydroxyl, $C_1$-$C_4$ alkanoyloxy, halo, halo-$C_1$-$C_4$ alkyl, -N($C_1$-$C_4$ alkyl)$_2$, -N$(CH_2)_m$,

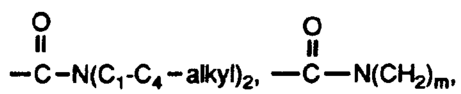

cyano, ethylenedioxy, benzyl, phenyl, or phenyl substituted by from 1 to 3 substituents selected from nitro, halo, $C_1$-$C_4$-alkyl, $C_1$-$C_4$ alkoxy, hydroxy, amino, or mono- or di-($C_1$-$C_4$ alkyl)amino;

m is an interger from 4 through 7;

$R^1$ is

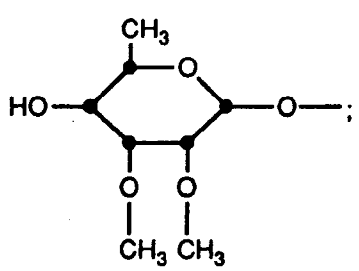 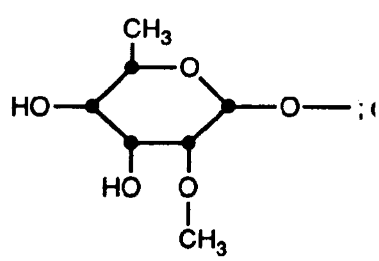

or

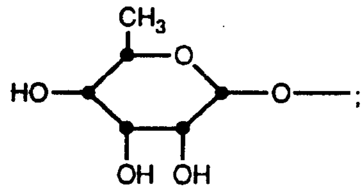

$R^2$ is hydrogen; $C_1$-$C_5$-alkanoyl or $C_1$-$C_5$-alkanoyl having from one to three halo substituents; benzoyl, phenylacetyl or phenylpropionyl or benzoyl, phenylacetyl or phenylpropionyl having from one to five halo or methyl or from one to two methoxyl, nitro or hydroxyl substituents;

$R^3$ is hydroxy; $C_1$-$C_5$ alkanoyloxy; $C_1$-$C_5$-alkanoyloxy having from one to three halo substituents; benzoyloxy, phenylacetoxy or phenoxyacetoxy or benzoyloxy, phenylacetoxy or phenoxyacetoxy having from one to five halo or methyl or from one to two methoxyl, nitro or hydroxyl substituents; or

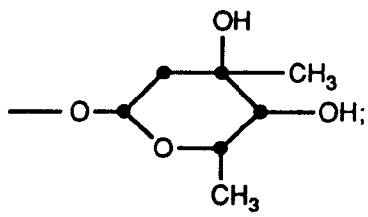

or an acid addition salt thereof;
in the preparation of a subcutaneous medicament useful for treating or preventing mastitis in a pregnant female ruminant.

To obtain the maximum benefits of the present invention, while diminishing residual levels of tilmicosin, the administration is timed with respect to the expected parturition. The administration should be not less than 25 days prior to parturition and preferably not less than 30 days prior to parturition. This time period will assure both efficacious treatment of the mastitis as well as the disappearance of the tilmicosin from the animal by the time of freshening.

The present invention is illustrated by the following examples.

## Example 1: Preparation of Formulation

A quantity of tilmicosin was slurried in water, the pH was adjusted to 6 by the addition of phosphoric acid, and propylene glycol was acided. The resulting formulation had the following formula:

| | |
|---|---|
| tilmicosin | 300 mg/mL |
| propylene glycol | 250 mg/mL |

This formulation is suitable to be used in treating mastitis in accordance with the present invention.

## Example 2

Field isolates of microorganisms causing mastitis were obtained; tilmicosin was evaluated in vitro against these microorganisms. Many of the microorganisms were known to be resistant to antibiotics. Three species of Staphylococcus aureus were resistant to both ampicillin and penicillin, and several Staphlylococcus agalactiae, and Staphylococcus uberis species were resistant to streptomycin and triple sulfa.

The in vitro evaluation of tilmicosin was done in a standard broth dilution test, with the following results.

## TABLE 1

### In vitro Susceptibility of Mastitis Pathogens

| Microorganism | No. Isolates | Cumulative No. With Various MIC (μg/mL) 0.195 | 0.39 | 0.78 | 1.56 | 3.12 | 6.25 | >50 |
|---|---|---|---|---|---|---|---|---|
| Staphylococcus aureus | (24) | 0 | 2 | 14 | 24 | -- | -- | |
| Streptococcus agalactiae | (10) | 0 | 3 | 8 | 10 | -- | -- | |
| Streptococcus dysgalactiae | (10) | 5 | 7 | 10 | -- | -- | -- | |
| Streptococcus uberis | (10) | -- | -- | -- | 2 | 5 | 10 | |

Example 3: Administration of Tilmicosin to Heifers

Nine heifers which had been bred and were due to calve in two months were employed in this experiment. They were maintained in a dry lot, fed a nonmedicated bred heifer ration, and allowed free access to fresh water. Each received a single 10 mg/kg injection in the dorsolateral thoracic area with the formulation of Example 1. Milk and serum samples were collected from each heifer at the first milking following parturition, and then at 12, 24, 36, 48, 60, 72, 84, 96, 108, 120, 132, 144, 156, and 168 hours later. Tilmicosin activity was determined in milk and serum samples using a microbiological assay with Micrococcus lutea (ATCC 9341) as the test organism. No tilmicosin activity was detected in any of the serum samples. Milk tilmicosin activity and calving intervals are listed in Table 2.

TABLE 2

Milk Tilmicosin Activity (μg/mL) in Heifers Following a Single 10 mg/kg Subcutaneous Injection 34-60 Days Prior to Calving

| Time (hr) | Animal Number 7776 | 7749 | 7747 | 7735 | 7761 | 7736 | 7729 | 7745 | 7794 |
|---|---|---|---|---|---|---|---|---|---|
| (1st milk) | 0.07 | 0.27 | 0.36 | 0.05 | 0.08 | 0.06 | 0.22 | 0.02 | 0.06 |
| 12 | 0.02 | 0.06 | 0.16 | 0.03 | NA | 0.03 | 0.23 | 0.02 | 0.03 |
| 24 | NA | 0.03 | 0.06 | 0.01 | NA | 0.01 | 0.21 | 0.04 | NA |
| 36 | NA | 0.03 | 0.04 | NA | NA | NA | 0.10 | 0.04 | NA |
| 48 | NA | 0.03 | 0.03 | NA | NA | NA | 0.07 | 0.02 | NS |
| 60 | NA | 0.01 | 0.02 | NA | NA | NA | 0.04 | 0.03 | NS |
| 72 | NA | NS | 0.02 | NA | NA | NA | 0.03 | 0.02 | NS |
| 84 | NA | 0.01 | 0.01 | NA | NA | NA | 0.03 | NS | NS |
| 96 | NA | 0.01 | NA | NA | NA | NA | 0.02 | NS | NS |
| 108 | NA | 0.01 | NA | NA | NA | NA | 0.02 | NS | NS |
| 120 | NA | NS | NA | NA | NA | NA | 0.02 | NS | NS |
| 132 | NA | 0.01 | NA | NA | NA | NA | 0.01 | NS | NS |
| 144 | NA | 0.01 | NA | NA | NA | NA | 0.02 | NS | NS |
| 156 | NA | NA | NA | NA | NA | NA | 0.01 | NS | NS |
| 168 | NA | NA | NA | NA | NA | NA | 0.01 | NS | NS |
| Treatment Interval[1] | 44 | 35 | 34 | 58 | 60 | 50 | 46 | 45 | 51 |

NA - No Activity
NS - No Sample
[1] Interval (days) from treatment to calving

Example 4: Administration of Tilmicosin at Drying Off

Nine Holstein dairy cows in late lactation were used for this study. Cows were fed an unmedicated complete dairy ration and had free access to fresh water. At drying off, all nine cows received a single 5 mg/kg subcutaneous injection with the formulation of Example 1 and groups of three cows each were assigned to withdrawal times of 24, 72, and 120 hours. At each withdrawal period, a milk sample was obtained from each quarter in all cows. Cows were then sacrificed, and a sample of udder tissue from each quarter was collected from all cows. Tilmicosin levels in milk and udder tissue were determined using an HPLC assay method. Control milk and udder tissue were used to generate standard curves. Individual milk and mammary tissue tilmicosin levels are listed in Tables 3 and 4, respectively. With only few exceptions, the milk and mammary tissue tilmicosin levels were relatively consistent in the four quarters for each cow.

Mean values for each of the three groups were also determined. Mean concentration of tilmicosin in milk (μg/mL) was 4.30, 2.16, and 1.85, while the mean concentration of tilmicosin in mammary tissue (μg/g) was 3.57, 3.34, and 5.97, for the 24, 72, and 120 hour withdrawals, respectively. The ratios of mean tilmicosin in mammary tissue to mean milk tilmicosin at 24, 72, and 120 hours after treatment were 0.83, 1.55, and 3.23, which indicates that during this period, tilmicosin persists to a greater degree in mammary tissue compared to milk. Both milk and mammary tissue levels were higher than the tilmicosin levels suggested by Example 2 for inhibiting growth of nearly all of the isolates tested.

## TABLE 3

### Milk Tilmicosin Concentration (μg/mL)

| Animal Number | Withdrawal (hr) | Mammary Quarter Sampled RF | RR | LF | LR | Mean | S.D. |
|---|---|---|---|---|---|---|---|
| 727 | 24 | 4.21 | 5.84 | 4.67 | 5.16 | 4.97 | 0.70 |
| 400 | 24 | 4.90 | 3.77 | 3.86 | 4.58 | 4.28 | 0.55 |
| 754 | 24 | 3.16 | 4.29 | 3.46 | 3.63 | 3.64 | 0.48 |
| | | | | | | 4.30 | 0.67 |
| 407 | 72 | 2.07 | 2.27 | 1.74 | 1.87 | 1.99 | 0.23 |
| 760 | 72 | 3.84 | 3.58 | 2.73 | 2.42 | 3.14 | 0.68 |
| 390 | 72 | 1.34 | 1.21 | 1.41 | 1.48 | 1.36 | 0.12 |
| | | | | | | 2.16 | 0.90 |
| 386 | 120 | 0.66 | 1.84 | 0.97 | 0.82 | 1.07 | 0.53 |
| 789 | 120 | 2.46 | 2.66 | 2.46 | 2.50 | 2.52 | 0.10 |
| 770 | 120 | 0.55 | 2.71 | 1.83 | 2.71 | 1.95 | 1.02 |
| | | | | | | 1.85 | 0.73 |

## TABLE 4

### Mammary Tissue Tilmicosin Concentration (μg/g)

| Animal Number | Withdrawal (hr) | Mammary Quarter Sampled RF | RR | LF | LR | Mean | S.D. |
|---|---|---|---|---|---|---|---|
| 727 | 24 | 4.38 | 3.99 | 3.87 | 4.66 | 4.23 | 0.36 |
| 400 | 24 | 2.58 | 2.69 | 4.12 | 3.61 | 3.25 | 0.74 |
| 754 | 24 | 3.75 | 2.75 | 3.10 | 3.27 | 3.22 | 0.42 |
| | | | | | | 3.57 | 0.57 |
| 407 | 72 | 3.55 | 2.90 | 3.71 | 1.99 | 3.04 | 0.78 |
| 760 | 72 | 0.51 | 4.26 | 6.12 | 1.67 | 3.14 | 2.53 |
| 390 | 72 | 4.80 | 3.26 | 3.68 | 3.68 | 3.85 | 0.66 |
| | | | | | | 3.34 | 0.44 |
| 386 | 120 | 3.15 | 5.47 | 3.94 | 3.77 | 4.08 | 0.99 |
| 789 | 120 | 10.50 | 6.50 | 11.20 | 5.79 | 8.50 | 2.75 |
| 770 | 120 | 2.63 | 5.34 | 7.37 | 6.03 | 5.34 | 2.00 |
| | | | | | | 5.97 | 2.28 |

Example 5: Administration of Tilmicosin to Mastitic Cows at Drying Off

The present invention was evaluated further in dairy cows entering the dry period, which were diagnosed as having mastitis attributable to Staphylococcus aureus in one or more quarters. The animals so identified were placed in five different groups; in one group, the animals were left untreated, to serve as a control. In another group, the animals were treated by conventional therapy, that is, by teat infusion with Cefa-Dri®: 300 mg cephapirin, Ayerst: The remaining three groups were treated in accordance with the present invention, that is, a formulation of tilmicosin prepared in accordance with Example 1 was subcutaneously administered; the doses of tilmicosin so administered varied among the three groups, being 2 mg/kg, 4 mg/kg, or 8 mg/kg. All animals were thereafter maintained under typical dry cow conditions until after parturition, at which time all animals were evaluated for mastitis due to Staphylococcus aureus. The results were as follows:

| Treatment | Number of quarters infected | Number of quarters cured |
|---|---|---|
| None (Control) | 1 | 0 |
| Cefa-Dri: 300 mg cephapirin | 5 | 3 |
| tilmicosin | | |
| 2 mg/kg | 1 | 0 |
| 4 mg/kg | 7 | 4 |
| 8 mg/kg | 5 | 3 |

Thus, of a total of 13 quarters infected with Staphylococcus aureus, treatment in accordance with the present invention cured 7 quarters.

## Claims

1. The use of a compound of formula I

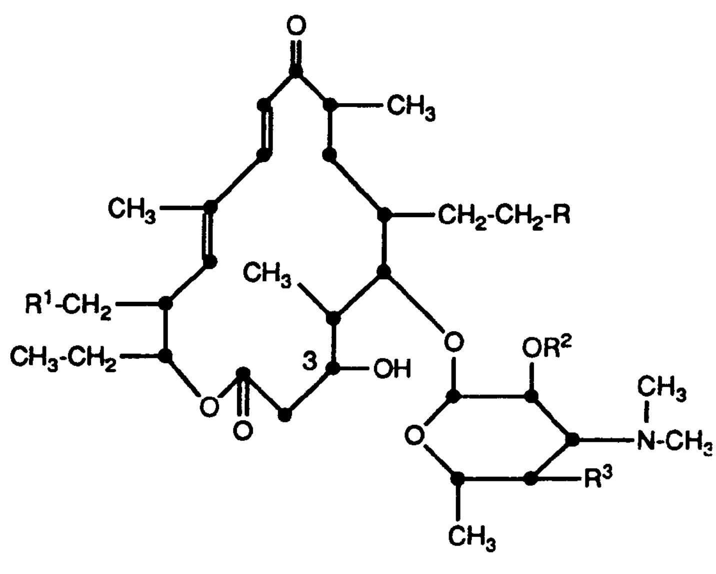

wherein

R is a saturated or unsaturated secondary amino group of the formula

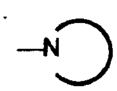

in which the nitrogen atom is part of an otherwise carbocyclic ring system selected from a monocyclic ring containing from 5 to 16 ring atoms or a bicyclic or tricyclic ring system containing from 8 to 20 ring atoms or such a group wherein one or more of the carbon atoms is substituted by $C_1$-$C_4$ alkyl, $C_2$-$C_4$ alkenyl, $C_2$-$C_4$ alkynyl, $C_1$-$C_4$ alkoxy, $C_1$-$C_4$ alkoxycarbonyl, hydroxyl, $C_1$-$C_4$ alkanoyloxy, halo, halo-$C_1$-$C_4$ alkyl, $-N(C_1$-$C_4$ alkyl$)_2$, $-N(CH_2)_m$,

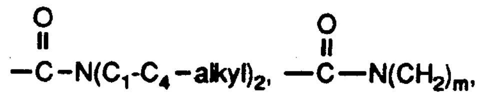

cyano, ethylenedioxy, benzyl, phenyl, or phenyl substituted by from 1 to 3 substituents selected from nitro, halo, $C_1$-$C_4$-alkyl, $C_1$-$C_4$ alkoxy, hydroxy, amino, or mono- or di-($C_1$-$C_4$ alkyl)amino;

m is an interger from 4 through 7;

$R^1$ is

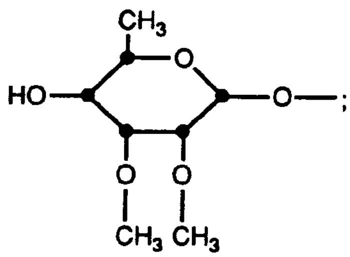

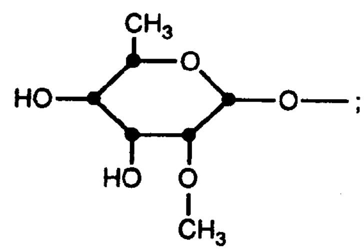

or

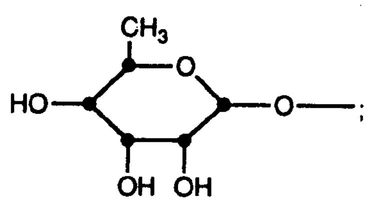

$R^2$ is hydrogen; $C_1$-$C_5$-alkanoyl or $C_1$-$C_5$-alkanoyl having from one to three halo substituents; benzoyl, phenylacetyl or phenylpropionyl or benzoyl, phenylacetyl or phenylpropionyl having from one to five halo or methyl or from one to two methoxyl, nitro or hydroxyl substituents;

$R^3$ is hydroxy; $C_1$-$C_5$ alkanoyloxy; $C_1$-$C_5$-alkanoyloxy having from one to three halo substituents; benzoyloxy, phenylacetoxy or phenoxyacetoxy or benzoyloxy, phenylacetoxy or phenoxyacetoxy having from one to five halo or methyl or from one to two methoxyl, nitro or hydroxyl substituents; or

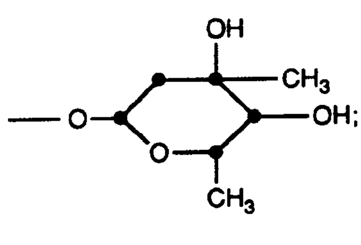

or an acid addition salt thereof;

in the preparation of a subcutaneous medicament useful for treating or preventing mastitis in a pregnant female ruminant.

2. The use of tilmicosin in the preparation of a medicament useful for treating or preventing mastitis in a pregnant female ruminant.

3. The use according to Claim 1 or 2, wherein the pregnant female ruminant is a lactating ruminant, and is thereafter maintained until parturition without further milking.

4. The use according to any one of Claims **1** to **3**, wherein the medicament is designed to be administered to the pregnant lactating ruminant not less than 25 days prior to parturition.

5. The use according to Claim **1** or **2** wherein the medicament is adapted for administration to a nulliparous heifer, said administration being carried out not less than 25 days prior to parturition.

European Patent Office

# EUROPEAN SEARCH REPORT

Application Number

EP 92 30 4005
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | DIALOG INFORMATION SERVICES FILE NO. 5: BIOSIS 1969-1992 ACCESSION NO. 8369164 J.H. WATTS et al.: 'Minimum inhibitory and bactericidal concentrations of seven antibiotics with bovine mastitis pathogens' & 91st General Meeting of the American Society for Microbiology 1991, Dallas, Texas, USA, May 5-9, 1991 --- | 1-5 | A61K31/365 A61K31/70 |
| Y | THE JOURNAL OF ANTIBIOTICS vol. 42, no. 8, August 1989, pages 1253 - 1267; M. DEBONO ET AL.: 'Synthesis and antimicrobial evaluation of 20-deoxo-20(3,5-dimethylpiperidin-1-yl)desmycosin (Tilmicosin, EL-870) and related cyclic amino derivatives' * page 1253 - page 1255; tables 3-4 * --- | 1-5 | |
| Y | THE JOURNAL OF ANTIBIOTICS vol. 40, no. 2, February 1987, pages 190 - 194; E.E. OSE: 'In Vitro Antibacterial Properties of EL-870, A New Semi-Synthetic Macrolide Antibiotic' * tables 1-3 * --- | 1-5 | TECHNICAL FIELDS SEARCHED (Int. Cl.5) A61K |
| Y | EPIDEM. INF. vol. 98, no. 3, 1987, pages 369 - 378; J. BALL ET AL.: 'Mycoplasma californicum mastitis in ewes as an experimental model for antibiotic treatment' * tables 1-2 * --- | 1-5 | |
| D,Y | US-A-4 820 695 (M. DEBONO) * column 14 - column 20; tables IX-XIII * --- -/-- | 1-5 | |

The present search report has been drawn up for all claims

| Place of search | Date of completion of the search | Examiner |
|---|---|---|
| MUNICH | 28 JULY 1992 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 92 30 4005
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | THE VETERINARY RECORD<br>vol. 128, 23 March 1991,<br>pages 278 - 280;<br>F.J. SCHUMANN: 'Prophylactic medication of feedlot calves with tilmicosin'<br>----- | | |
| | | | TECHNICAL FIELDS SEARCHED (Int. Cl.5 ) |

The present search report has been drawn up for all claims

| Place of search | Date of completion of the search | Examiner |
|---|---|---|
| MUNICH | 28 JULY 1992 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)